# EUROPEAN PATENT SPECIFICATION

(11) **EP 0 497 820 B1**
(45) Date of publication and mention of the grant of the patent: **12.06.1996**
(21) Application number: 90915660.6
(22) Date of filing: 16.10.1990
(51) Int. Cl.: A61K 31/70, A61K 38/55

(54) **RIBOZYME INHIBITORS**
RIBOZYMENHEMMER
INHIBITEURS DE RIBOZYMES

(30) Priority: 26.10.1989 US 427707
(43) Date of publication of application: 12.08.1992
(73) Proprietor: UNIVERSITY OF COLORADO FOUNDATION, INC., Boulder, CO 80306 (US)
(72) Inventor: CECH, Thomas, R., Boulder, CO 80306 (US)
(74) Representative: Sexton, Jane Helen
(86) International application number: US9005945
(87) International publication number: WO9106302

(56) References cited:
- US-A- 4 654 326
- PROCEEDINGS OF THE NATIONAL ACADEMY OF SCIENCES OF USA, vol. 85, no. 2, January 1988, WASHINGTON, US, pages 519-523; KUHN, R.J. ET AL.: 'Construction of amutagenesis cartridge for poliovirus genome-linked viral protein: isolation andcharacterization of viable and nonviable mutants'
- PROCEEDINGS OF THE NATIONAL ACADEMY OF SCIENCES OF USA, vol. 86, no. 6, March 1989, WASHINGTON, US, pages 1831-1835; WU, H.-N. ET AL.: 'Human hepatitis delta virus RNA subfragments contain an autocleavage activity'
- EMBO JOURNAL, vol. 7, no. 8, 1988, EYNSHAM, OXFORD, GB, pages 2523-2532; MUNROE, S.H.: 'Antisense RNA inhibits splicing of pre-mRNA in vitro'
- TRENDS IN BIOTECHNOLOGY, vol. 8, no. 7, July 1990, CAMBRIDGE, GB, pages 174-178; COTTEN, M.: 'The in vivo application of ribozymes'
- Molecular and Cellular Biology, Vol. 9, No. 10, October 1989; COTTEN et al.: "Ribozyme, Antisense RNA, and Antisense DNA Inhibition of U7 Small Nuclear Ribonucleoprotein-Mediated Histone Pre-mRNA Processing In Vitro", pp. 4479-4487
- Biochemistry, Vol. 28, No. 3, 07 February 1989; YARUS: "Specificity of Arginine Binding by the Tetrahymena Intron", pp. 980-988
- Science, Vol. 231, 31 January 1986; ZARG and CECH: "The Intervening Sequence RNA of Tetrahymena Is an Enzyme", pp. 470-475, Fig. 5
- Biochemistry, Vol. 25, No. 16, 12 August 1986; BASS and CECH: "Ribozyme Inhibitors: Deoxyguanosine and Dideoxyguanosine Are Competitive Inhibitors of Self-Slicing of the Tetrhymena Ribosomal Ribonucleic Acid Precursor", pp. 4473-4477

## Description

### Background of the Invention

This invention concerns inhibitors of the biological activity of a class of RNA molecules called ribozymes.

Ribozymes are RNA molecules that have a biological activity that may be intramolecular, that is, the RNA can self-splice or self-cleave, or intermolecular, that is, the RNA has an enzymatic activity, such as an endoribonuclease activity that is active to cleave another RNA molecule. Kruger et al., 31 Cell 147, 1982; Zaug et al., 324 Nature 429, 1986; Zaug and Cech, 231 Science 470, 1986; and Guerrier-Takada et al., 35 Cell, 849, 1983.

Bass and Cech, 25 Biochemistry 4473, 1986, describe in vitro competitive inhibitors of a ribozyme of Tetrahymena thermophila. These inhibitors compete for the guanosine substrate site of the ribozyme, and include deoxyguanosine and dideoxyguanosine.

Cech and Bass, 55 Annual Review Biochemistry 599, 1986, and Zaug and Cech, 231 Science 470, 1986, describe deoxy C₅ as an in vitro competitive inhibitor of an intermolecular reaction between a ribozyme and the homopentanucleotide C₅.

Tanner and Cech, 13 Nucleic Acid Research 7741, 1985, and Tanner and Cech, 13 Nucleic Acid Research 7759, 1985 describe in vitro inhibition of the self-catalyzed cyclization of the IVS RNA of Tetrahymena by small molecules including ethidium bromide and methidiumpropyl EDTA. Spermidine and magnesium chloride have some effect on the inhibitory action of one or more of these compounds.

### Summary of the Invention

Applicant has discovered a class of nucleic acids and amino acid based compounds that interact specifically with, and in some cases bind tightly to, ribozymes in vivo. Such interaction causes significant inhibition of the biological activity of those ribozymes. These inhibitors are useful as prophylactic and therapeutic agents, since they can be used for treatment, or prevention, of infection of an animal or plant with organisms (which term includes fungi, viruses or other infectious nucleic acids such as viroids, virusoids and satellite RNAs) that depend for a part of their lifecycle upon the biological activity of a ribozyme. The inhibitory molecules may be introduced into an animal or plant by any of several standard techniques, as described below.

According to the invention there is provided the use of a pharmacologically acceptable nucleic acid or amino acid based compound that interacts specifically with a ribozyme in the manufacture of a medicament for use in a method for reducing the in vivo effect on an animal of infection with an organism having a ribozyme with a biological activity, comprising the steps of:
(a) providing a pharmacologically acceptable nucleic acid or amino acid based compound that interacts specifically with the ribozyme,
(b) introducing said nucleic acid or amino acid based compound into the animal in a manner to allow contact of said nucleic acid or amino acid based compound with the ribozyme, whereby the biological activity of the ribozyme is reduced.

According to the invention there is further provided a method for reducing the in vivo effect on a plant of infection with an organism having a ribozyme with a biological activity, comprising the steps of:
(a) providing a pharmacologically acceptable nucleic acid or amino acid based compound that interacts specifically with the ribozyme.
(b) introducing said nucleic acid or amino acid based compound into the plant in a manner to allow contact of said nucleic acid or amino acid based compound with the ribozyme, whereby the biological activity of the ribozyme is reduced.

In application the invention provides a method for reducing the in vivo effect on an animal, e.g., a mammal, such as a human, or a plant; e.g., avocado, of infection with an organism having a ribozyme with a biological activity. The method includes providing a nucleic acid or amino acid based compound that interacts specifically with the ribozyme. The nucleic acid or amino acid based compound is then introduced into the animal or plant in a manner to allow contact of the nucleic acid or amino acid based compound with the ribozyme, whereby the biological activity of the ribozyme is reduced or eliminated.

As discussed above, ribozymes include any self-splicing or self-cleaving RNA found within an organism. Generally, such ribozymes have the ability, in the absence of any protein, to cause themselves to be cleaved into two or more -A fragments. In addition, a ribozyme may have other biological activities, e.g., enzymatic activities, including the ability to cause attachment of polypeptides to RNA molecules. Examples of such ribozymes include those associated with various plant infectious agents, viroids and virusoids (Prody et al., Science 1577, 1986; Hutchins et al., 14 Nuc. Acid. Res. 3627, 1986; Forster et al., 49 Cell 211, and 50 Cell 9, 1987). In these organisms a ribozyme catalyzes self-cleavage, and perhaps ligation, of RNA as a step that appears to be critical in the replication cycle of the infectious RNA. In some of these organisms, for example poliovirus, there is a ribozyme molecule that participates in addition of a small virus-encoded polypeptide, VPg, to the 5' end of the minus strand RNA. This polypeptide is required for productive replication of the RNA. Other Picornaviruses, including rhinoviruses, foot-and-mouth disease virus, and hepatitis A virus are similar to poliovirus in that they have a small polypeptide attached to the 5'-end of their virion and minus-strand RNAs. This addition of VPg appears to be ribozyme catalyzed in poliovirus (and probably in other picornaviruses) and thus susceptible to inhibitors of the present invention. As another example, hepatitis delta virus, Sharmeen et al., 62 J. of Virol. 2674, 1988, undergoes self-cleavage in a manner similar to the plant virusoids, and participates in the attachment of a protein (the hepatitis delta viral antigen) to itself. Another example is a ribozyme present in Pneumocystis carinii, a fungus that infects man (Sogin and Edman, 17 Nucl. Acids Res. 5349, 1989). It is a self-splicing precursor RNA, which is homologous to the ribozyme of Tetrahymena; this self-splicing is required for biosynthesis of ribosomal RNA, and thus for protein synthesis. Fungal mitochondria are abundant sources of self-splicing ribozymes; thus, clinically important fungi, such as Monila and Candida, may potentially contain mitochondrial or even nuclear self-splicing introns. These examples are not limiting in this invention; any small, generally circular, RNA molecule that is associated with a virus or any viral RNA covalently attached to a polypeptide is potentially a ribozyme. Sharmeen et al., supra.

Nucleic acid molecules useful in the present invention are generally molecules formed of ribo- or deoxyribo-nucleotides, but may be formed as a mixture of ribonucleotides and deoxyribonucleotides, or analogs of ribo- or deoxyribo-nucleotides. Preferably, they are at least 6-40, most preferably 8-20 nucleotides in length. They also include nucleic acid molecules that encode an inhibitory molecule, for example, a DNA molecule encoding an inhibitory RNA molecule. Such DNA molecules may be introduced by any standard technique into an animal or plant and caused to express the inhibitory RNA molecule in a manner that causes the RNA molecule to contact the ribozyme to be inhibited. Nucleic acid molecules useful in the invention include nucleic acids which are derivatized or modified to inhibit the nucleic acid from undergoing cleavage or splicing by a ribozyme.

Amino acid-based compounds useful in this invention are those proteins, polypeptides, peptides, peptide analogs (a peptide having one or more chemical groups not normally present in a peptide), or non-peptide mimics of polypeptides that specifically inhibit or reduce the biological activity of a ribozyme. Such amino acid based compounds include those that specifically compete with a polypeptide necessary for a ribozyme reaction, for example, the VPg polypeptide of a poliovirus. They also include cyclic and conformationally restricted peptides and analogues formed in vitro, especially those able to enter a cell or other structure containing a ribozyme. Those skilled in the art will recognize that the active portion of the polypeptide (see, e.g., Kuhn et al., 85 Proc. Natl. Acad. Sci. USA, 519, 1988.), or the site of the ribozyme that recognizes this polypeptide, can be readily identified and a synthetic amino acid-based compound or polypeptide, or a genetically engineered recombinant peptide or polypeptide, may be synthesized in vitro or in vivo and used in this invention as an inhibitor.

Inhibitors useful in this invention include nucleic acid molecules having high complementarity to a biologically active site (or binding site) of a ribozyme, such that they strongly bind at the active site and reduce biological activity of the ribozyme. Active sites of ribozymes may be identified and characterized by any standard technique, as described by Been and Cech, 47 Cell 207, 1986, Cech, 73 Gene 259, 1988, Davies et al., 300 Nature 719, 1982; Forster and Symons, 49 Cell 211, 1987; Forster and Symons, 50 Cell 9, 1987; Forster et al., 334 Nature 265 1988; Hampel and Tritz, 28 Biochemistry 4929, 1989; Haseloff and Gerlach, 334 Nature 585, 1988; Waring et al., 321 Nature 133, 1986; and Wu et al., 86 Proc. Natl. Acad. Sci. U.S.A. 1831,1989. Once the nucleotide sequence of the active site is known, inhibitory nucleic acid molecules are readily synthesized and tested for their inhibitory activity on the active site.

The high complementarity that is desired in this invention provides specificity of reaction of an inhibitor with a ribozyme. Thus, an inhibitor will generally only bind specifically with a ribozyme to be inhibited, and not with any other molecule. Usually the inhibitor binds tightly with the ribozyme, having a Kd of less than 10 nM, preferably less than 1nM. Even if another molecule is bound, it is unlikely that such binding will have any appreciable or significant effect on the animal being treated. Thus, inhibitors of this invention are extremely specific and are expected to have few or no side effects on the organism being treated.

Introduction of the inhibitory molecule into an animal may be by any of several techniques, including oral or direct administration of the inhibitor into the bloodstream or a specific organ of an animal, or by injection of RNA within or nearby any specific cell of that animal. In addition, as mentioned above, inhibitory nucleic acid may be synthesized in vivo, e.g., from DNA encoding that nucleic acid introduced to an animal by transfection, or an equivalent technique, to introduce that DNA molecule into a cell under the control of a desired promotor or other regulatory region. Introduction of inhibitory amino acid based compounds is by any standard technique, including direct administration of a therapeutically effective amount to an animal or plant, or by introduction of nucleic acid which can cause production of such an amino acid based compound.

In a related aspect the invention features a therapeutic composition that is a mixture of a pharmaceutically acceptable carrier or vehicle, e.g., a buffer, and an inhibitory nucleic acid molecule or amino acid based compound that binds specifically with a ribozyme present within an animal. Generally, the composition includes a therapeutically effective amount of such an inhibitory compound.

Other features and advantages of the invention will be apparent from the following description of the preferred embodiments, and from the claims.

### Description of the Preferred Embodiments

The drawings will first briefly be described.

### Drawings

Fig. 1 is a diagrammatic representation of the predicted folded structure of a ribozyme of T. thermophila showing the locations of a G-binding site ("G-site"), a 5' exon-binding site, and a core site ("Core"); nucleotides are numbered and various paired regions (P) and loops (L) labelled according to standard nomenclature;
Fig. 1a is a diagrammatic representation of the G-binding site of a T. thermophila ribozyme;
Fig. 2a is a diagrammatic representation of the folded structure of the P9 and P10 regions of a T. thermophila ribozyme; the G-binding site is shown as a rectangle, in this case binding a G residue attached to the 3' exon of the ribozyme;
Figs. 2b and c show inhibitors (in bold) that block interaction of the T. thermophila ribozyme with itself, and thus prevent self-splicing; xG is a G nucleotide modified to render it inactive in cleavage, e.g., deoxy G;
Fig. 3 is a diagrammatic representation of the 5' exon-binding site of a T. thermophila ribozyme showing normal self-splicing (A), and inhibition (i.e., the ribozyme reacts with the inhibitor instead of the substrate leading to inactivation of the ribozyme) of such splicing by an inhibitor (B);
Figs. 4a, 4b and 4c are diagrammatic representations of the predicted folded structure of a ribozyme of Hepatitis Delta Virus; nucleotides are marked according to the nomenclature of Wu et al. supra;
Figs. 4b and 4c show the inhibitory action of RNA molecules (in bold) complementary to nucleotide bases 679-695 (Fig. 4b) or homologous (Fig. 4c) to nucleotide bases 680-695;
Figs. 5a and 5b are diagrammatic representations of monomeric and dimeric forms respectively of Avocado Sunblotch Viroid RNA (ASBV) showing a hammerhead-type structure. The self-cleavage sites are shown by arrows. Bases shown in blocks are conserved in various ribozymes of this type;
Figs. 5c and 5d show two modes of inactivation by inhibitors having the sequences shown in bold. Inhibition of splicing is shown by crossed arrows.

### Inhibitory Molecules

Nucleic acid and amino acid based compounds that are suitable as inhibitors in this invention are generally discussed above. Here, Applicant presents a few examples of inhibitory molecules and methods by which others can be identified. This description is provided merely as an example and is not limiting in this invention. Those skilled in the art will recognize that many other inhibitory molecules can be designed using equivalent techniques.

One inhibitor of this invention is a RNA molecule having the ribonucleotide sequence CCCUCU. This inhibitor is active as an inhibitor of the ribozyme of T. thermophila, characterized by Kruger et al., supra. Any other nucleic acid, polypeptide, or derivative thereof (i.e., a modified nucleic acid or polypeptide), that binds to and/or reacts or interacts (e.g., by hydrogen bonding or ionic interactions) with a portion of a ribozyme, or reacts or interacts with a ribozyme and its natural biological substrate (which may be another portion of the same ribozyme molecule, or a polypeptide, such as the VPg polypeptide of poliovirus RNA) is potentially useful in this invention. Such interaction of an inhibitor with a ribozyme will inactivate the ribozyme, or prevent or reduce its reaction with its natural substrate (including interaction with itself). By inactivating or reducing the biological activity of a ribozyme the infectivity of the virus, or other biological entity or organism containing or encoding the ribozyme, is reduced or abolished.

Generally, in order to determine whether a given nucleic acid is useful in the invention, the structure, composition and sequence of the reactive site of a ribozyme is first determined by standard procedure, and a nucleic acid molecule having a nucleotide base sequence complementary to the reactive site of the ribozyme, or to any other biologically active domain of the ribozyme (examples of which are provided below) is synthesized. The ribozyme and the inhibitor are then mixed in vitro to determine whether the inhibitor inhibits a biological activity, preferably an essential biological activity, e.g., self splicing, of the ribozyme.

The administration method involves introducing the inhibitor to infected cells or tissue using any appropriate delivery system. Such systems include liposome mediated fusion with a target cell, causing direct uptake of the inhibitor by the target cells by simply contacting the cell with the inhibitor, or transfection of the inhibitor into cells followed by reintroduction of the cells into a patient. Small peptides, or analogs thereof, may be administered orally or by intravenous or intramuscular injection. The inhibitor may act by any number of means, including acting as a competitive inhibitor that resembles the naturally occurring ribozyme substrate; acting as a substrate but giving an inactive product that is of no use to the organism containing the ribozyme; and as an inhibitor that has a nucleotide base sequence complementary to the active site of a ribozyme, and binds to the active site in a manner that denatures the structure required for a biological activity of the ribozyme.

Having now described the general format of this invention, Applicant will provide examples that will clarify the specific issues concerned with different classes of ribozymes. These examples include strategies for inhibition of the biological activity of four ribozymes, which illustrate the broad scope of the invention. The invention is not limited to these specific examples; most of the strategies described are applicable to all four systems and other related systems, even if mentioned for only one. Ribozymes other than those explicitly discussed are also susceptible to inhibition by inhibitors identified as described below, or by equivalent procedures.

The first example is a ribozyme belonging to a class called "group I introns." The specific example is the T. thermophila pre-rRNA intron. The close similarity in splicing mechanism within the group I intron group makes it clear to one of ordinary skill in the art that this example applies equally to any other group I intron, with modification being necessary only to account for a particular nucleotide sequence of any chosen ribozyme. The second example, HDV RNA (a human pathogen), is included to show how inhibitors can be readily identified for a ribozyme whose structure and mechanism of action are unknown. The third example, a ribozyme found in a plant viral satellite RNA, is included to show the generality of this invention. The fourth example concerns poliovirus RNA and is different from the other examples in that a polypeptide-RNA combination is involved. The example presented demonstrates how one of ordinary skill in the art can readily devise inhibitors of this class of ribozyme, and then use them in the invention. Poliovirus is a picornavirus, and analogous strategies are applicable to other picornaviruses (e.g., rhinoviruses, foot-and-mouth disease virus), to non-picornaviruses in which a covalent protein-RNA linkage is formed, and to other related ribozyme-containing organisms.

### Example 1: Group I intron

Group I intron RNA folds into a complex 3-dimensional structure to form a biologically active center to accomplish RNA self-splicing. Cech, 236 Science 1532, 1987, and Cech, 73 Gene 259, 1988. Group I introns found in RNAs from diverse biologic sources (including fungal mitochondria and nuclei, plant chloroplasts, and bacteriophage) share the same fundamental catalytic structure, and accomplish splicing by the same fundamental mechanism. Thus, mechanistic features established for one group I intron, such as that of the T. thermophila nuclear pre-rRNA, are generally applicable to all group I introns. For example, all group I intron splicing mechanisms involve a guanosine or GTP molecule that is bound by the intron and acts as a nucleophile, cleaving the 5' splice site. RNA splicing is an essential step in gene expression, so interfering with splicing in a living organism will inactivate the gene containing the group I intron.

The inhibitor may be designed to act at one of several sites on the ribozyme. These sites include a G-binding site, a 5' exon-binding site, and a core of the ribozyme. Examples of each are now provided.

### G-binding site

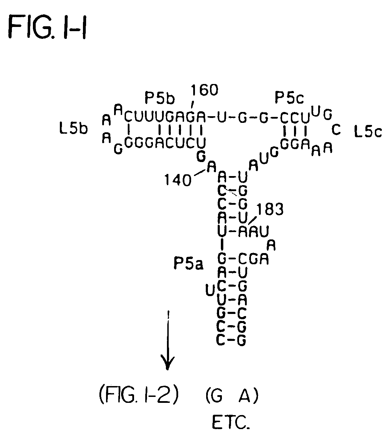

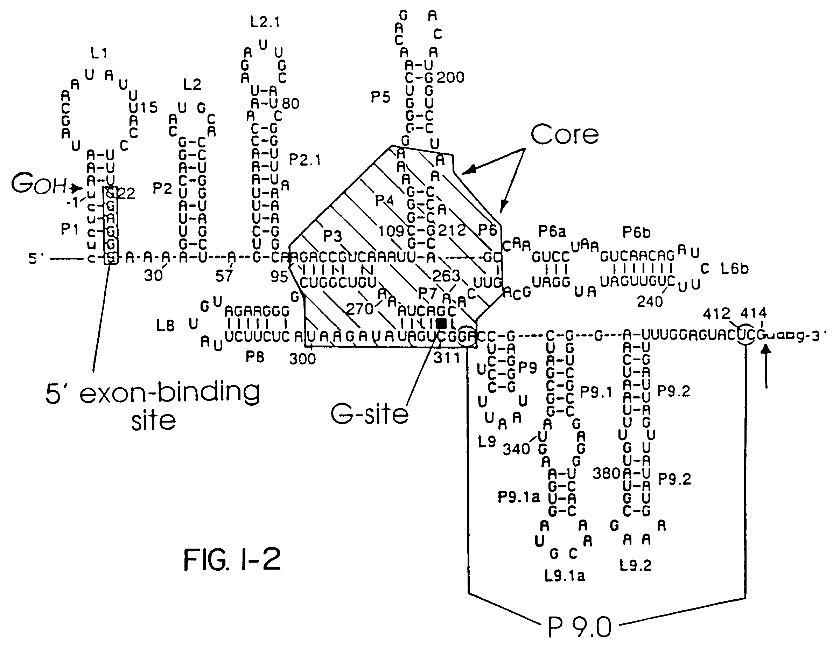

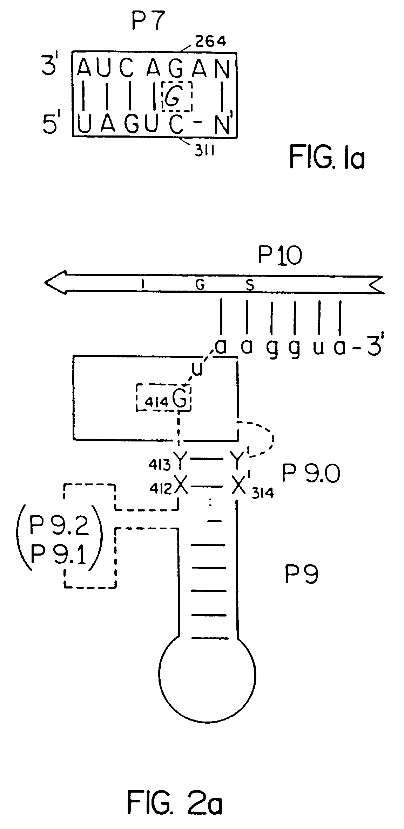

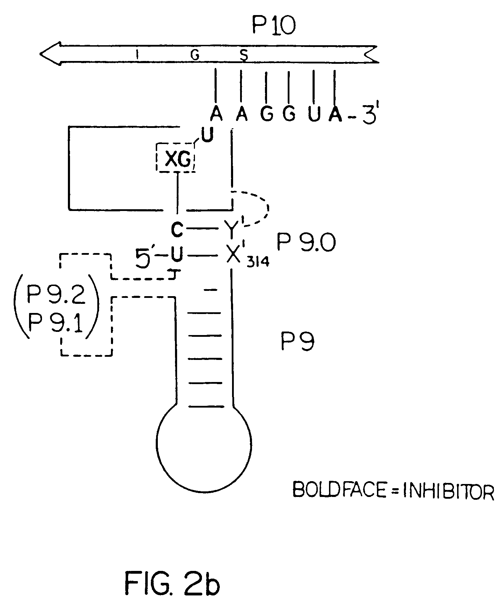

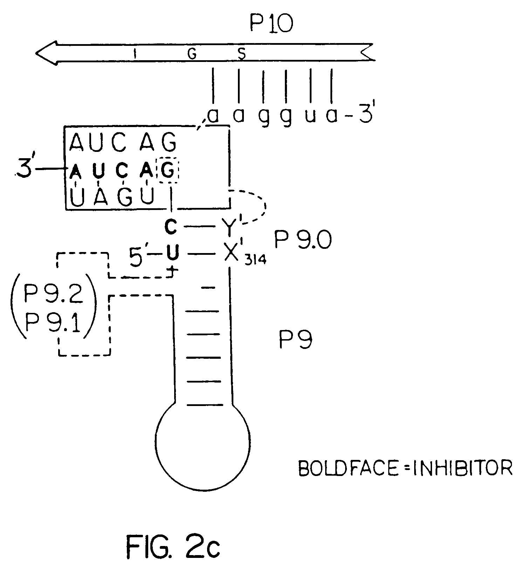

Referring to Figs. 1 and 1a, the G-site of the Tetrahymena ribozyme is shown. Nucleotides near the 3' end of the intron (marked "3'") preceding the conserved G (number 414) are involved in binding interactions at the G-site. Inhibitory nucleic acid can be synthesized that is homologous in nucleotide base sequence to this 3' region in the Tetrahymena intron. For example, UC(xG), a trinucleotide with the same sequence as the last 3 nucleotides of the intron (see Fig. 1, nucleotides 412-414) but with an unreactive xG replacing the reactive ribo-G could be used. xG is guanosine with a modification of the ribose sugar that prevents it acting as a nucleophile (for example, 3'-deoxy G, or 2'-deoxy G). Another set of inhibitors includes those of the general form UC(xG)UAAGGUA, which extends the trinucleotide sequence into the 3' exon. These inhibitors act on the structure shown in Fig. 2a. The predicted binding mode of an inhibitor of this class is shown in Fig. 2b. Yet another set of inhibitors includes those of the general formula UCGACUA..., where the dots represent the possibility that continuing the sequence would make an even better inhibitor. Here, the inhibitor includes the above trinucleotide sequence (UCG) that is bound by the G-site, and also includes an adjacent sequence (ACUA...) that binds to and displaces one half of the functionally critical P7 stem (Fig. 1a). The predicted binding mode of an inhibitor of this class is shown in Fig. 2c. It is clear to one of ordinary skill in this art that other equivalent inhibitors can be devised that bind at the G-binding site of any desired group I intron, and optionally at another site, thereby preventing, competitively or otherwise, interaction of that site with its natural substrate, namely guanosine.

### 5' exon-binding site

### (i) Inhibition of Splicing

The 5' exon-binding site of the ribozyme is a portion of the internal guide sequence and binds the last few nucleotides at the 5' exon by Watson-Crick base-pairing (Fig. 1). Additional interactions also help hold the 5' exon nucleotides in place (Sullivan & Cech, 42 Cell 639, 1985). The sequence near the 5' splice site of the Tetrahymena pre-rRNA is CUCUCU/AAA, (Fig. 1, shown at P1 adjacent the G_{OH}-site; the slash represents the splice site). Applicant has found that an oligonucleotide containing the related sequence, CCCUCUAAA, binds to the intron with a K_{d} of about 1 nM. Replacement of ribo- with deoxyribo-nucleotides does not prevent binding, but prevents reaction. Thus, inhibitors of the first step of splicing (reaction at the 5' splice site) include molecules of the type CCCUC(dT)(dA)AA, where the 5' splice site is bounded by deoxyribonucleotides.

Although dissociation constants about 1 nM are quite low they may be inadequate; thus, an even tighter-binding inhibitor can be synthesized by extending the length of the inhibitor nucleotide sequence into the L1 domain while maintaining complementarity with the intron: e.g., CCCUC(dT)(dA)AAGGUAA..., or by derivatizing the oligonucleotide to increase its binding constant, e.g., by the covalent addition of an ethidium bromide molecule to a terminal nucleotide.

Inhibitors having a sequence complementary to any other group I intron 5' exon-binding site and optionally modified as described above to include a longer sequence of complementary nucleotides, and/or to incorporate molecules such as ethidium, are also useful in this invention.

### (ii) Promotion of Mis-Splicing

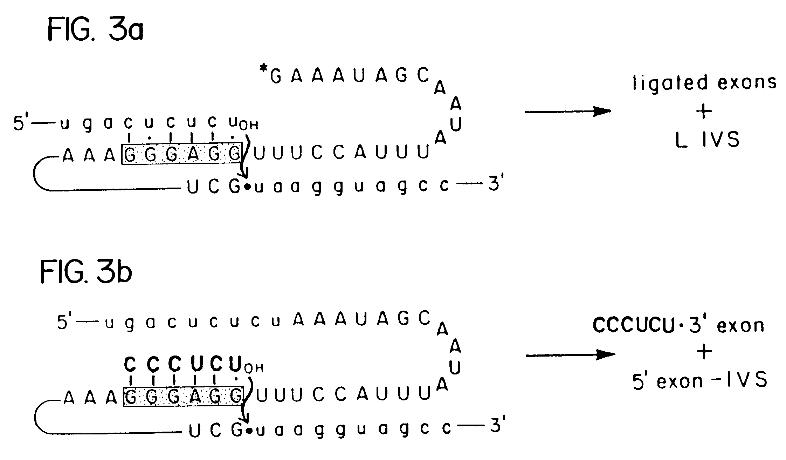

One limitation of the competitive inhibitors discussed above is their inherent reversibility. Whenever the inhibitor is released, splicing can proceed. In the following example, splicing occurs with an exogenous oligonucleotide instead of an endogenous 5' exon, thereby breaking the precursor RNA into two molecules and destroying it. Oligonucleotides able to make 6 base-pairs with the internal guide sequence, e.g., one ending with the sequence CCCUCU, are tight-binding substrate analogues (K_{d} approximately the same as that of oligonucleotides containing CCCUCUAAA, described above). Thus, administration of the hexanucleotide CCCUCU, or derivatives thereof (e.g., derivatives containing deoxy C residues, or including nucleotides having ethidium incorporated therein) that retain a free 3'-terminal hydroxyl group, can promote intermolecular exon ligation (i.e., ligation of two RNA molecules), as shown in Fig. 3. This reaction short-circuits the biological activity of a ribozyme and inactivates that activity. Substrate homologs of other group I introns are also useful in this manner.

### Ribozyme Core

The exogenous oligonucleotides described above can displace their endogenous counterparts in the 5' splice-site region as described above. The same displacement reaction can be generally applied to other regions, e.g., the core region, of the intron and thereby cause inhibition of biological activity. By synthesizing and administering an exogenous oligonucleotide composed of RNA or something other than RNA (e.g., DNA or a copolymer of RNA and DNA), a new pairing can be formed that in general is stable but non-functional. For example, a deoxyoligonucleotide complementary to positions 257-274 of the Tetrahymena intron can bind thereto and thereby interfere with the formation of the P3, P6 and P7 structural elements (Fig. 1), which are essential for self-splicing activity. Thus, such molecules will be useful in this invention.

### Example 2: Hepatitis delta virus (HDV) RNA

Hepatitis B virus, which causes viral hepatitis and hepatocellular carcinoma in man, has a circular, partially double-stranded DNA genome. HDV is a satellite virus of hepatitis B virus that contains a 1700 nucleotide circular RNA genome. HDV can superinfect carriers of hepatitis B virus to cause an extremely severe, fulminant hepatitis. Sharmeen et al., supra, report that HDV RNA undergoes site-specific self-cleavage in vitro, producing 2', 3'-cyclic phosphate and 5'-hydroxyl termini. Self-cleavage is thought to be an essential step in the replication of the HDV RNA; thus, inhibiting this step is likely to be an effective therapeutic strategy against infection by this virus.

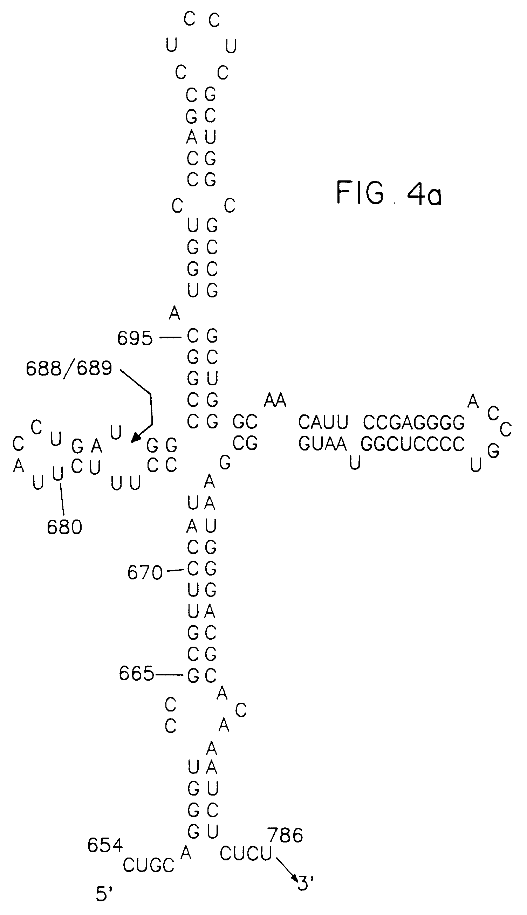

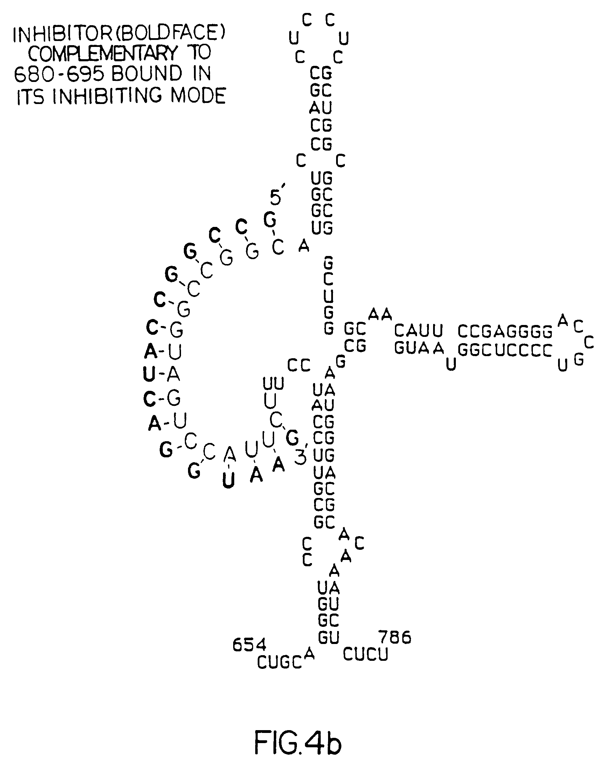

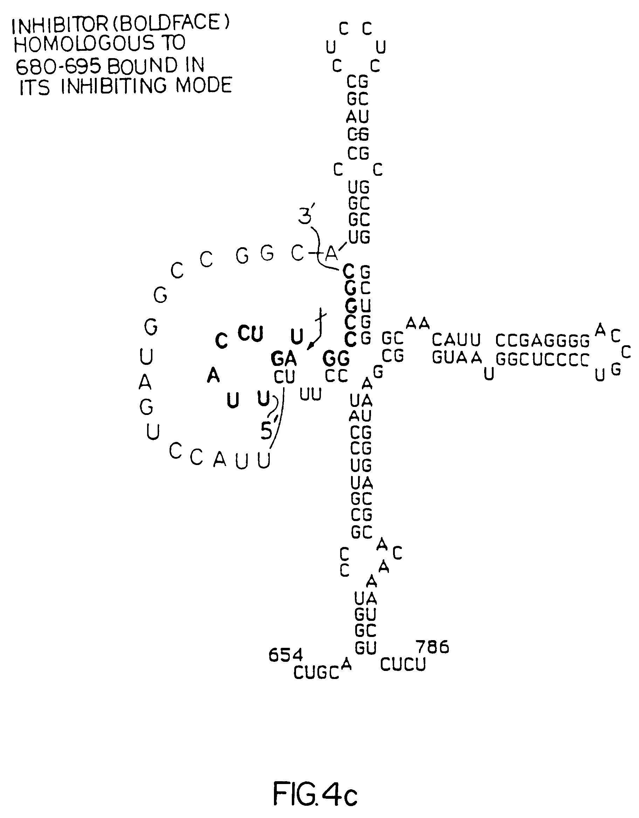

Wu et al. supra, identify the region responsible for HDV RNA self-cleavage as within the 133 nucleotides shown in Fig. 4a. The secondary structure shown in Fig. 4 is computer-generated, and its validity is not to be assumed.

To define the smallest oligonucleotide that binds well to HDV RNA and inhibits self-cleavage, the first step is to synthesize moderate-size DNA and RNA molecules that are complementary to and homologous to sequences around the self-cleavage site (around bases 665-695). One set of such DNA and RNA molecules (inhibitors) has the sequence of nucleotide positions 665-695 (Fig. 4a) and the complement of that sequence.

The second step is to determine the initial velocity of self-cleavage (Vₒ) as a function of inhibitor concentration, thereby deriving an I₅₀ (concentration of inhibitor required to decrease V₀ by 50%) for each oligonucleotide. If I₅₀ is much less than 1 nM, then smaller versions can be synthesized to define the smallest useful inhibitor, i.e., one retaining an I₅₀ of less than 1nM. In the present example, oligonucleotides representing positions 665-680 and 680-695 can be tested. If, for example, 680-695 is found to be a viable inhibitor, 680-690 and 687-690 can be tested. The mode of inhibition of two such inhibitors is shown in Figs. 4b and c for RNA complementary (Fig. 4b) or homologous (Fig. 4c) to nucleotides 680-695. If the I₅₀ is greater than 1 nM, then larger oligonucleotides can be tested, and derivatization with intercalating agents, such as ethidium bromide, or other DNA-binding agents, undertaken to increase the binding ability of the oligonucleotide. These in vitro studies will provide a basic characterization of the system. Useful inhibitors identified by this or equivalent methods can then be used in vivo.

It is important to ascertain the mode of inhibition on an oligoribonucleotide inhibitor, i.e., to determine whether the inhibitor is cleaved. This is easily ascertained by incubation of radiolabeled inhibitor with an excess concentration of HDV RNA. If cleaved, the inhibitor can be made more efficacious by preventing cleavage. One of the following strategies should prove successful. First, a phosphorothioate linkage is introduced at the cleavage site; both the Sₚ and Rₚ isomers can be tested. Second, one or two deoxyribonucleotides are introduced at positions flanking the cleavage site. In each case, a decrease in I₅₀ indicates an improved inhibitor.

Other inhibitors active on these types of viral RNAs can be identified by this technique. One must simply identify a potential ribozyme, determine the nucleotide sequence of active portions of the ribozyme, and then synthesize inhibitors having complementary nucleotide sequences to the ribozyme.

### Example 3: Plant viral satellite, virusoid and viroid RNAs

Viroids are small circular RNAs that are infectious by themselves in plants. Virusoids and satellite RNAs are circular and linear forms, respectively, that are encapsulated by coat proteins of certain plant RNA viruses. Many such RNAs undergo efficient site-specific self-cleavage in vitro. During infection, self-cleavage is thought to convert a linear multimeric RNA replication intermediate into unit-sized products. Inhibiting self-cleavage is likely to inhibit replication of the virus.

Most of the RNAs share a small structural domain, containing about 30 nucleotides that form 3 helical arms. This "hammerhead" structure is necessary and sufficient for self-cleavage, e.g., in Avocado sunblotch viroid (ASBV, Uhlenbeck, 308 Nature 596, 1987; Forster et al., 50 Cell 9, 1987).

A ribozyme related to these hammerhead types is the 359 nucleotide negative strand satellite RNA of tobacco ringspot virus that undergoes efficient self-cleavage, but does not contain a hammerhead motif. Hampel and Tritz, 28 Biochemistry 4929, 1989 describe the biologically active domain contained within a 50 nucleotide sequence, which efficiently cleaves a RNA substrate containing 14 nucleotides of satellite RNA sequence.

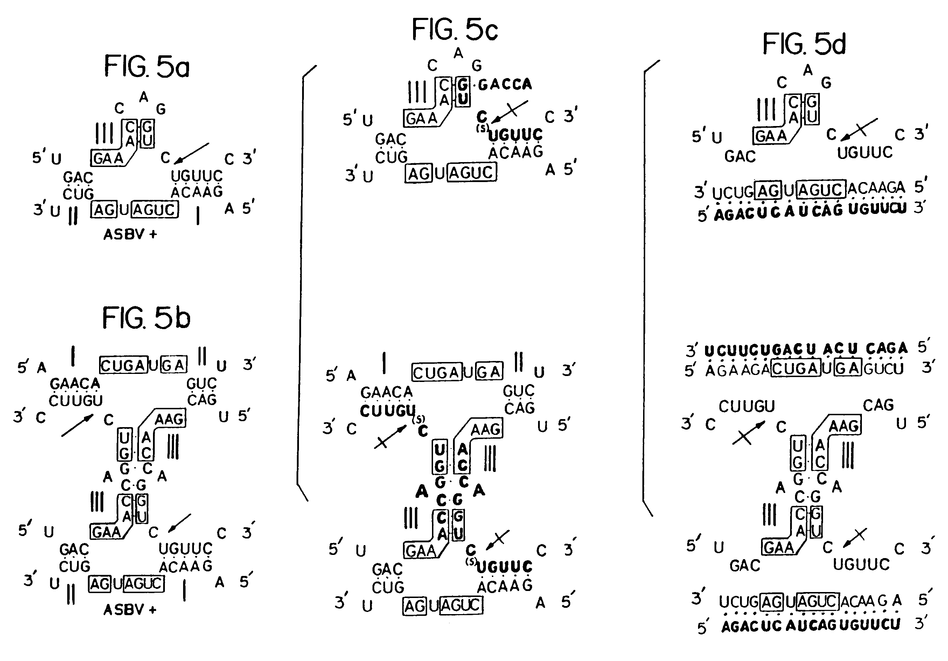

The sequence of ASBV flanking the self-cleavage site is ACCAGGUC/UGUUC (Fig. 5a, b). In analogy to the inhibitors proposed above, the following inhibitors of ASBV RNA self-cleavage are proposed: d(ACCAGGTCTGTTC), ACCAGGU(dC)(dT)GUUC, and ACCAGGUC(s)UGUUC where (s) represents a phosphorothioate substitution at the cleavage site. The proposed binding mode of one such inhibitor is shown in Fig. 5c. The activity of these inhibitors can be tested by determination of I₅₀, and those that show specific inhibition will be shortened, e.g., d(GTCTGTTC), or derivatized to enhance binding as described above.

Formation of the catalytic core of the "hammerhead" requires that the sequence 5'-CUGANGA-3' (boxed in the bottom strand of Fig. 5a and in the top and the bottom strand of Fig. 5b) not be in a double helix so that it is available for tertiary structure formation. Oligonucleotides of the type AGACTCATCAGTGTTCT, complementary to the bottom strand of Fig. 5a and to the bottom and top strand in Fig. 5b, can base pair to it and thereby prevent or disrupt formation of the biologically active center (Fig. 5d).

While the inhibitors described above are designed to inhibit ASBV, simply changing the nucleotide sequence of the inhibitor to match that of the infectious RNA allows one of ordinary skill in the art to extend the methodology to any satellite or virusoid RNA with a defined self-cleavage domain.

### Example 4: Picornaviral RNAs

Poliovirus RNA has a virus-encoded polypeptide (VPg) covalently linked to the 5' ends of both the positive strand (virion RNA) and the negative strand (which acts as a template during RNA replication). Poliovirus appears to use RNA catalysis to accomplish VPg attachment. VPg attachment in vitro has minimal requirements: chemically-synthesized VPg, a deproteinized viral RNA replication intermediate, and MgCl₂. In this simple system, cleavage takes place to produce the 5' end of the negative strand covalently linked to VPg. The reaction occurs by transesterification, with the hydroxyl group of a tyrosine of VPg serving as the nucleophile, much as the 3'-hydroxyl of guanosine serves as the nucleophile for group I intron self-splicing. It appears that the viral RNA is the catalyst for addition of VPg. Even if VPg attachment was to turn out not to be RNA-catalyzed in the strict sense, it is clearly RNA-mediated, and therefore susceptible to inhibition in vivo. VPg attachment is essential for viral replication, so any inhibitor of the process is likely to inhibit viral proliferation.

Since the poliovirus RNA sequences responsible for mediating VPg attachment have not been identified, the first step is to localize such sequences. This is done by the standard method of making a series of deleted versions of the RNA to identify the critical region (see, e.g., Haseloff and Gerlach, 334 Nature 585, 1988). A series of oligonucleotides of moderate length (20-30 nucleotides each) homologous to the viral positive-strand are synthesized to span the region implicated in directing VPg attachment. A second series of oligonucleotides complementary to the viral positive-strand are also synthesized. Both series of oligonucleotides are tested as inhibitors in the in vitro system described above. The strategy then follows that described in the above examples; the smallest effective inhibitory oligonucleotide is derivatized to increase its binding constant to RNA, its hydrophobicity, and its resistance to nuclease degradation. Each derivative is then tested for inhibition of VPg attachment in vitro and for decreased viability of poliovirus in vivo.

Polypeptides, peptides, peptide analogues, and appropriate non-peptide ligands (peptide mimetics) can be used as inhibitors. There are at least two general approaches to the design of such inhibitors. In a first, the structure of VPg is analyzed by NMR spectroscopy or X-ray crystallography, and organic non-peptide compounds synthesized to mimic the three-dimensional structure of the amino-terminal portion of VPg. This portion contains the tyrosine residue responsible for attachment of VPg to RNA. VPg is a small polypeptide and might form a stable structure only in the presence of the RNA with which it interacts; if this is the case, the structure of the RNA-VPg complex is determined. Methods for synthesis of conformationally restricted nonpeptide mimetics of various protein structure motifs are described by Kahn et al., 110 J. Am. Chem. Soc. 1638, 1988; Kahn et al., 1 J. Mol. Recog. 75, 1988; Kemp and Sites, 29 Tet. Lett. 5057, 1988; Kemp and Curran 29 Tet. Lett. 4931, 1988; Kemp and Carter, 28 Tet. Lett. 4641, 1987; Kemp and Bowen, 29 Tet. Lett. 5077, and 5081, 1988; Garcia-Echeverria et al., 30 Tet. Lett. 2441, 1989; Krstenansky et al., 108 J. Am. Chem. Soc. 1696, 1986; and Hruby 8 Trends in Pharm. Sci. 336, 1987.

In a second approach, conformationally restricted peptide-like compounds are synthesized to mimic the amino acid sequence of a desired region of VPg without knowledge of VPg structure. These are then empirically tested for ability to inhibit virus replication in tissue culture cells. Kuhn et al., 85 Proc. Nat. Acad. Sci. USA 519, 1988, and 62 J. Virol. 4207, 1988. The region of VPg mimicked is preferably the first five amino acids of the protein. The basis for this choice can be seen in the Table below. The first 5 amino acids are more conserved in sequence among picornaviruses than the remainder of the protein, and this region includes the essential tyrosine residue (Y) at position 3.

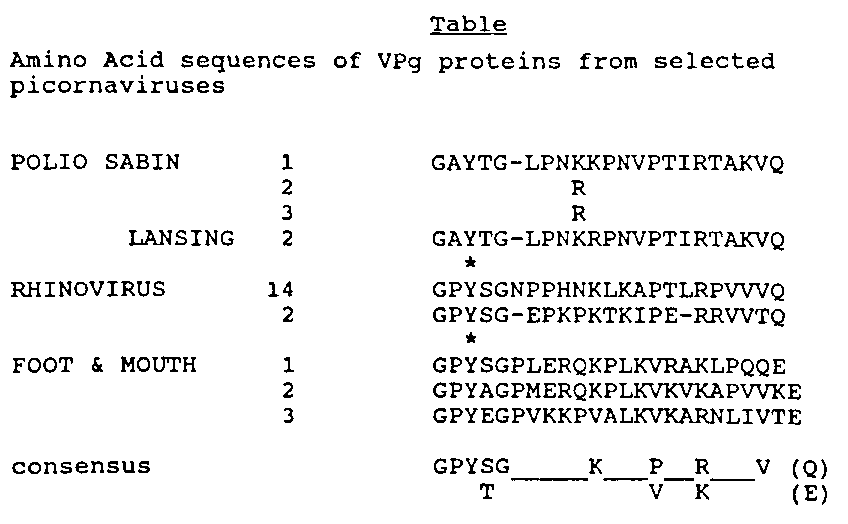

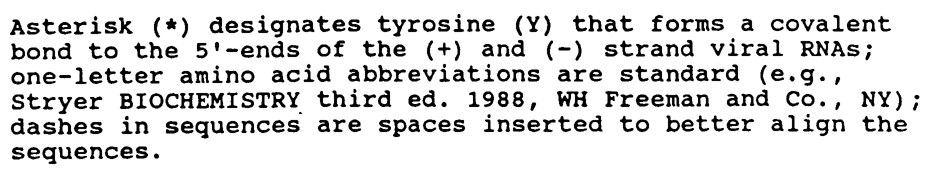

Design considerations include the following: (1) the peptide preferably has low molecular weight (<1000 and preferably <500) and substantial hydrophobicity to enable it to enter cells readily; (2) the peptide preferably is conformationally restricted or "rigid" to give tight binding to the RNA; (3) the peptide preferably is stable against degradation in vivo to give good biological activity; and (4) the peptide preferably is a competitive inhibitor of VPg; e.g., containing a phe instead of tyr at position 3 so that the peptide is inactive in the reaction; or the peptide can be synthesized with a phe in position 3, allowing reaction of the peptide with the RNA but giving a product that is unable to continue the infection cycle. The following are nonlimiting examples of such peptides:

### Cyclic peptides

Cyclic peptide versions of the first five amino acids are useful, e.g., of amino acid sequence GAYTG and GAFTG for poliovirus, and GPYSG and GPFSG for rhinovirus and foot-and-mouth disease virus (see Table above). Related sequences are readily chosen in analogous manner for other picornaviruses. The replacement of F for Y at position 3 may allow binding but prevent nucleophilic attack by the peptide, since F lacks the nucleophilic hydroxyl group present in of Y. Thus, such peptides are preferred in this invention. Other derivatives are also useful, e.g., cyclic peptide disulfides, such as Ac-C-P-Y-C-NH₂ and Ac-C-P-F-C-NH₂, where the two C (cysteine) residues form the disulfide. These can be synthesized as described by Garcia-Echeverria et al., supra.

### Dipeptide mimetic

Dipeptide mimetics based on lactams, thiazoles, imidazolines and other cyclic moieties (Morgan and Gainor, 24 Ann. Rep. Med. Chem. 243, 1989) are incorporated into small peptide-related compounds, for example GPY (lactam) G, shown below, or GPF (lactam) G. These peptides mimic the first 5 amino acids of VPg but are expected to be inactive with the ribozyme.

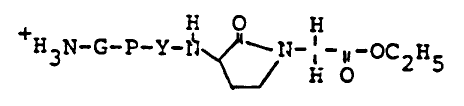

The compounds described above can be derivatizied such that one or more basic, e.g., amine functionalities are incorporated at either end of the compound. This takes advantage of the fact that VPg's typically contain several basic amino acid residues (K or R, in Table above) which are thought to facilitate binding to RNA (Kuhn et al., J. Virol., supra). Short peptides can also be synthesized which have the active site of the VPg-type molecules, e.g., the first 5-10 amino acids, such that they can interact with a ribozyme but fail to provide an active product.

### Methods

As discussed above the inhibitors used in this invention can be synthesized and administered by any standard procedure, dependent upon the structure of the inhibitor. Examples of such methods are briefly described below.

### Inhibitors composed strictly of RNA

Some of the proposed inhibitors consist of underivatized RNA, and can be produced in vivo by introducing, into living cells, a DNA vector that encodes the RNA. DNA oligonucleotides encoding the two strands of the RNA inhibitor can be made on an automated DNA synthesizer, annealed to form a double-stranded unit, and ligated into an appropriate vector adjacent to an appropriate promoter. An appropriate promoter includes one that directs transcriptional initiation by a RNA polymerase found in the cells to be treated (e.g., human or plant RNA polymerase I, II or III).

The recombinant DNA is then cloned in, e.g., E.coli, transcribed in vitro to confirm that it is capable of directing the transcription of an active inhibitor, and introduced into an animal by standard transgenic techniques, including microinjection, or by transfection into cells. Alternatively, a nonvirulent, recombinant form of a virus such as adenovirus or vaccinia virus encoding the ribozyme inhibitor in its viral genome can be produced; upon infection of the human or other animal, the cellular polymerases will transcribe the ribozyme inhibitor. For plants, RNA can be encoded in a vector such as the Ti plasmid of Agrobacterium tumefaciens that is transfected into plant cells. In all cases, transcription of the recombinant DNA by cellular or viral RNA polymerase produces the desired RNA molecule, which then inhibits cellular ribozymes, or the ribozymes of infecting viruses or viral-associated RNAs.

### Inhibitors composed of DNA, DNA-RNA copolymers, and derivatives thereof

These inhibitors can be synthesized in vitro and subsequently introduced into cells.

DNA can be directly synthesized on an automated DNA synthesizer such as Applied Biosystems Model 380A. RNA oligonucleotides also can be directly synthesized on such an instrument. A large number of base, ribose and phosphate modifications can be incorporated during chemical synthesis by substituting appropriate reagents for the normal phosphoramadites. Alternatively, RNA and derivatives containing modified nucleotides (e.g., deoxyribonucleotides) or modified phosphodiester linkages (e.g., phosphorothioate diesters) can be synthesized by transcription in vitro using synthetic DNA as template. An example in which a single phosphorothioate is incorporated into a specific position in an oligoribonucleotide is given in McSwiggen and Cech, 24 Science 679, 1989.

Small oligonucleotides are spontaneously taken up from the surrounding medium by some cells. Such uptake may be facilitated by modification of the nucleic acid such as derivatization with a hydrophobic moiety, substitution of methylphosphonates, phosphorothioates or dithioates for normal phosphates. Liposome fusion provides another mode of delivering nucleic acid-based reagents to cells. Generally, such inhibitors are provided at between 10 and 10,000 µg/kg of animal or plant to be treated; but can be provided at lower doses when applied locally to a specific tissue. These levels are therapeutically effective for reduction of ribozyme activity.

### Polypeptide Inhibitors

Administration of amino acid based compound inhibitors, such as those related to picornaviral VPg's follows the same strategies described for oligonucleotide-based inhibitors. The peptide mimetic and other polypeptides of low molecular weight and hydrophobic character will enter cells more easily; thus, oral administration or intravenous or intramuscular injection should be appropriate. Again, generally such inhibitors are provided at between 10 and 10,000 µg/kg of animal or plant to be treated. Alternatively, standard polypeptides may be synthesized in vivo from engineered nucleic acid introduced as described above, under the control of a desired promoter. Expression of the inhibitor can then be turned on by regulation of the promoter by standard procedure.

Other embodiments are within the following claims.

## Claims

1. Use of a pharmacologically acceptable nucleic acid or amino acid based compound that interacts specifically with a ribozyme in the manufacture of a medicament for use in a method for reducing the *in vivo* effect on an animal of infection with an organism having a ribozyme with a biological activity, comprising the steps of:
(a) providing a pharmacologically acceptable nucleic acid or amino acid based compound that interacts specifically with the ribozyme,
(b) introducing said nucleic acid or amino acid based compound into the animal in a manner to allow contact of said nucleic acid or amino acid based compound with the ribozyme, whereby the biological activity of the ribozyme is reduced.

2. Use according to claim 1 wherein step (a) comprises providing a pharmacologically acceptable nucleic acid based compound that interacts specifically with the ribozyme.

3. Use according to claim 1 or 2 wherein said nucleic acid molecule comprises ribonucleotides.

4. Use according to claim 1 or 2 wherein said nucleic acid comprises ribonucleotides derivatized or modified to inhibit said nucleic acid from undergoing cleavage or splicing by a ribozyme.

5. Use according to claim 1 or 2 wherein the biological activity is a self-cleaving or self-splicing activity.

6. Use according to claim 1 or 2 wherein the biological activity is attachment of a polypeptide to the ribozyme.

7. Use according to claim 1 or 2 wherein said nucleic acid binds tightly with the ribozyme.

8. Use according to claim 1 wherein said amino acid based compound attaches to said ribozyme.

9. A method for reducing the *in vivo* effect on a plant of infection with an organism having a ribozyme with a biological activity, comprising the steps of:
(a) providing a pharmacologically acceptable nucleic acid or amino acid based compound that interacts specifically with the ribozyme,
(b) introducing said nucleic acid or amino acid based compound into the plant in a manner to allow contact of said nucleic acid or amino acid based compound with the ribozyme, whereby the biological activity of the ribozyme is reduced.

10. A method according to claim 9 wherein step (a) comprises providing a pharmacologically acceptable nucleic acid based compound that interacts specifically with the ribozyme.

11. The method of claim 9 or 10 wherein said nucleic acid molecule comprises ribonucleotides.

12. The method of claim 9 or 10 wherein said nucleic acid comprises ribonucleotides derivatized or modified to inhibit said nucleic acid from undergoing cleavage or splicing by a ribozyme.

13. The method of claim 9 or 10 wherein the biological activity is a self-cleaving or self-splicing activity.

14. The method of claim 9 or 10 wherein the biological activity is attachment of a polypeptide to the ribozyme.

15. The method of claim 9 or 10 wherein said nucleic acid binds tightly with the ribozyme.

16. The method of claim 9 wherein said amino acid based compound attaches to said ribozyme.

17. A composition, comprising a nucleic acid or amino acid based compound that interacts specifically with a ribozyme reducing its biological activity which ribozyme is present within an animal or plant, said nucleic acid or amino acid based compound being admixed with a buffer.

18. A composition according to claim 17 which is a therapeutic composition wherein the ribozyme is present within an animal, the nucleic acid or amino acid based compound is pharmacologically acceptable and the buffer is a pharmaceutically acceptable buffer.

19. The composition of claim 17 or 18 wherein said nucleic acid molecule comprises ribonucleotides.

20. The composition of claim 17, 18 or 19 wherein the biological activity is a self-cleaving or self-splicing activity.

21. The composition of any one of claims 17 to 20 wherein said nucleic acid binds tightly with the ribozyme.

22. The composition of claim 17 or 18 wherein said nucleic acid comprises polynucleotides derivatized or modified to inhibit said nucleic acid from undergoing cleavage or splicing by a ribozyme.

23. The composition of claim 17 or 18 wherein the biological activity is attachment of a polypeptide to the ribozyme.

24. The composition of claim 17 or 18 wherein said amino acid based compound attaches to the ribozyme.

## Patentansprüche

1. Verwendung einer pharmakologisch annehmbaren Verbindung auf Nukleinsäure- oder Aminosäurebasis, welche spezifisch mit einem Ribozym interagiert, bei der Herstellung eines Medikamentes zum Einsatz in einem Verfahren zur Verminderung des *in vivo*-Effektes einer Infektion durch einen ein Ribozym mit einer biologischen Aktivität aufweisenden Organismus auf ein Tier, umfassend die folgenden Schritte:
(a) Vorsehen einer pharmakologisch annehmbaren Verbindung auf Nukleinsäure- oder Aminosäurebasis, welche spezifisch mit dem Ribozym interagiert,
(b) Einführen der Verbindung auf Nukleinsäure- oder Aminosäurebasis in das Tier auf eine Weise, welche den Kontakt der Verbindung auf Nukleinsäure- oder Aminosäurebasis mit dem Ribozym ermöglicht, wodurch die biologische Aktivität des Ribozyms verringert wird.

2. Verwendung nach Anspruch 1, worin der Schritt (a) das Vorsehen einer pharmakologisch annehmbaren Verbindung auf Nukleinsäurebasis, welche spezifisch mit dem Ribozym interagiert, umfaßt.

3. Verwendung nach Anspruch 1 oder 2, worin das Nukleinsäuremolekül Ribonukleotide umfaßt.

4. Verwendung nach Anspruch 1 oder 2, worin die Nukleinsäure Ribonukleotide umfaßt, welche derivatisiert oder modifiziert sind, um zu verhindern, daR die Nukleinsäure eine Spaltung oder ein Spleißen durch ein Ribozym erleidet.

5. Verwendung nach Anspruch 1 oder 2, worin die biologische Aktivität eine Selbst-Spaltungs- oder Selbst-Spleißaktivität ist.

6. Verwendung nach Anspruch 1 oder 2, worin die biologische Aktivität die Anlagerung eines Polypeptides an das Ribozym ist.

7. Verwendung nach Anspruch 1 oder 2, worin die Nukleinsäure fest mit dem Ribozym bindet.

8. Verwendung nach Anspruch 1, worin die Verbindung auf Aminosäurebasis sich an das Ribozym anlagert.

9. Verfahren zur Verminderung des *in vivo*-Effektes einer Infektion durch einen ein Ribozym mit einer biologischen Aktivität aufweisenden Organismus auf eine Pflanze, umfassend die folgenden Schritte:
(a) Vorsehen einer pharmakologisch annehmbaren Verbindung auf Nukleinsäure- oder Aminosäurebasis, welche spezifisch mit dem Ribozym interagiert,
(b) Einführen der Verbindung auf Nukleinsäure- oder Aminosäurebasis in die Pflanze auf eine Weise, welche den Kontakt der Verbindung auf Nukleinsäure- oder Aminosäurebasis mit dem Ribozym ermöglicht, wodurch die biologische Aktivität des Ribozyms verringert wird.

10. Verfahren von Anspruch 9, worin der Schritt (a) das Vorsehen einer pharmakologisch annehmbaren Verbindung auf Nukleinsäurebasis, welche spezifisch mit dem Ribozym interagiert, umfaßt.

11. Verfahren von Anspruch 9 oder 10, worin das Nukleinsäuremolekul Ribonukleotide umfaßt.

12. Verfahren von Anspruch 9 oder 10, worin die Nukleinsäure Ribonukleotide umfaßt, welche derivatisiert oder modifiziert sind, um zu verhindern, daR die Nukleinsäure eine Spaltung oder ein Spleißen durch ein Ribozym erleidet.

13. Verfahren von Anspruch 9 oder 10, worin die biologische Aktivität eine Selbst-Spaltungs- oder Selbst-Spleißaktivität ist.

14. Verfahren von Anspruch 9 oder 10, worin die biologische Aktivität die Anlagerung eines Polypeptides an das Ribozym ist.

15. Verfahren von Anspruch 9 oder 10, worin die Nukleinsäure fest mit dem Ribozym bindet.

16. Verfahren aus Anspruch 9, worin die Verbindung auf Aminosäurebasis sich an das Ribozym anlagert.

17. Zusammensetzung, umfassend eine Verbindung auf Nukleinsäure- oder Aminosäurebasis, welche spezifisch mit einem Ribozym interagiert, wodurch dessen biologische Aktivität vermindert wird, welches Ribozym innerhalb eines Tieres oder einer Pflanze vorhanden ist, wobei die Verbindung auf Nukleinsäure- oder Aminosäurebasis mit einem Puffer vermischt ist.

18. Zusammensetzung nach Anspruch 17, welche eine therapeutische Zusammensetzung ist, worin das Ribozym innerhalb eines Tieres vorhanden ist, die Verbindung auf Nukleinsäure- oder Aminosäurebasis pharmakologisch annnehmbar ist, und der Puffer ein pharmazeutisch annehmbarer Puffer ist.

19. Zusammensetzung von Anspruch 17 oder 18, worin das Nukleinsäuremolekül Ribonukleotide umfaßt.

20. Zusammensetzung von Anspruch 17, 18 oder 19, worin die biologische Aktivität eine Selbst-Spaltungs oder Selbst-Spleißaktivität ist.

21. Zusammensetzung aus mindestens einem der Ansprüche 17 bis 20, worin die Nukleinsäure fest mit dem Ribozym bindet.

22. Zusammensetzung von Anspruch 17 oder 18, worin die Nukleinsäure Polynukleotide umfaßt, welche derivatisiert oder modifiziert sind, um zu verhindern, daß die Nukleinsäure eine Spaltung oder ein Spleißen durch ein Ribozym erleidet.

23. Zusammensetzung von Anspruch 17 oder 18, worin die biologische Aktivität die Anlagerung eines Polypeptides an das Ribozym ist.

24. Zusammensetzung von Anspruch 17 oder 18, worin die Verbindung auf Aminosäurebasis sich an das Ribozym anlagert.

## Revendications

1. Utilisation d'un composé pharmacologiquement acceptable à base d'acide nucléique ou d'acides amidés, qui interagit spécifiquement avec un ribozyme, dans la fabrication d'un médicament pour utilisation dans une méthode de réduction de l'effet *in vivo* sur un animal d'une infection provoquée par un organisme ayant un ribozyme à activité biologique, comprenant les étapes consistant à :
(a) fournir un composé pharmacologiquement acceptable à base d'acide nucléique ou d'acides aminés qui interagit spécifiquement avec le ribozyme,
(b) introduire ledit composé à base d'acide nucléique ou d'acides aminés dans l'animal de manière à permettre le contact dudit composé à base d'acide nucléique ou d'acides aminés avec le ribozyme, ce qui entraîne une réduction de l'activité biologique du ribozyme.

2. Utilisation selon la revendication 1, dans laquelle l'étape (a) comprend la fourniture d'un composé pharmacologiquement acceptable à base d'acide nucléique qui interagit spécifiquement avec le ribozyme.

3. Utilisation selon la revendication 1 ou 2, dans laquelle ladite molécule d'acide nucléique comprend des ribonucléotides.

4. Utilisation selon la revendication 1 ou 2, dans laquelle ledit acide nucléique comprend des ribonucléotides, dérivés ou modifiés de manière à inhiber le clivage ou l'épissage dudit acide nucléique par un ribozyme.

5. Utilisation selon la revendication 1 ou 2, dans laquelle l'activité biologique est une activité d'auto-clivage ou d'auto-épissage.

6. Utilisation selon la revendication 1 ou 2, dans laquelle l'activité biologique est la fixation d'un polypeptide au ribozyme.

7. Utilisation selon la revendication 1 ou 2, dans laquelle ledit acide nucléique se lie solidement avec le ribozyme.

8. Utilisation selon la revendication 1, dans laquelle ledit composé à base d'acides aminés se fixe audit ribozyme.

9. Méthode de réduction de l'effet *in vivo* sur une plante d'une infection provoquée par un organisme ayant un ribozyme à activité biologique, comprenant les étapes consistant à :
(a) fournir un composé pharmacologiquement acceptable à base d'acide nucléique ou d'acides aminés qui interagit spécifiquement avec le ribozyme,
(b) introduire ledit composé à base d'acide nucléique ou d'acides aminés dans la plante de manière à permettre le contact dudit composé à base d'acide nucléique ou d'acides aminés avec le ribozyme, ce qui entraîne une réduction de l'activité biologique du ribozyme.

10. Méthode selon la revendication 9, dans laquelle l'étape (a) comprend la fourniture d'un composé pharmacologiquement acceptable à base d'acide nucléique qui interagit spécifiquement avec le ribozyme.

11. Méthode selon la revendication 9 ou 10, dans laquelle ladite molécule d'acide nucléique comprend des ribonucléotides.

12. Méthode selon la revendication 9 ou 10, dans laquelle ledit acide nucléique comprend des ribonucléotides, dérivés ou modifiés de manière à inhiber le clivage ou l'épissage dudit acide nucléique par un ribozyme.

13. Méthode selon la revendication 9 ou 10, dans laquelle l'activité biologique est une activité d'auto-clivage ou d'auto-épissage.

14. Méthode selon la revendication 9 ou 10, dans laquelle l'activité biologique est la fixation d'un polypeptide au ribozyme.

15. Méthode selon la revendication 9 ou 10, dans laquelle ledit acide nucléique se lie solidement avec le ribozyme.

16. Méthode selon la revendication 9, dans laquelle ledit composé à base d'acides aminés se fixe audit ribozyme.

17. Composition, comprenant un composé à base d'acide nucléique ou d'acides aminés qui interagit spécifiquement avec un ribozyme réduisant son activité biologique, le ribozyme étant présent dans un animal ou dans une plante, ledit composé à base d'acide nucléique ou d'acides aminés étant mélangé avec un tampon.

18. Composition selon la revendication 17, qui est une composition thérapeutique où le ribozyme est présent dans un animal, le composé à base d'acide nucléique ou d'acides aminés est pharmaceutiquement acceptable et le tampon est un tampon pharmaceutiquement acceptable.

19. Composition selon la revendication 17 ou 18, dans laquelle ladite molécule d'acide nucléique comprend des ribonucléotides.

20. Composition selon la revendication 17, 18 ou 19, dans laquelle l'activité biologique est une activité d'auto-clivage ou d'auto-épissage.

21. Composition selon l'une quelconque des revendications 17 à 20, dans laquelle ledit acide nucléique se lie solidement avec le ribozyme.

22. Composition selon la revendication 17 ou 18, dans laquelle ledit acide nucléique comprend des polynucléotides, dérivés ou modifiés de manière à inhiber le clivage ou l'épissage dudit acide nucléique par un ribozyme.

23. Composition selon la revendication 17 ou 18, dans laquelle l'activité biologique est la fixation d'un polypeptide au ribozyme.

24. Composition selon la revendication 17 ou 18, dans laquelle ledit composé à base d'acides aminés se fixe au ribozyme.
